# EUROPEAN PATENT APPLICATION

(11) **EP 4 578 937 A1**
(43) Date of publication of application: **02.07.2025**
(21) Application number: 24220502.9
(22) Date of filing: 17.12.2024
(51) Int. Cl.: C12M 1/12, C12M 1/26, C12M 1/00, C12N 1/02

(54) **APPARATUS AND METHOD FOR MICROORGANISM DETECTION INVOLVING FILTRATION**

(30) Priority: 27.12.2023 JP 2023221177
(71) Applicant: Yokogawa Electric Corporation, Tokyo 180-8750 (JP)
(72) Inventor: MITSUMORI, Yuuji, Musashino-shi, Tokyo 180-8750 (JP); TAGUCHI, Tomoyuki, Musashino-shi, Tokyo 180-8750 (JP)
(74) Representative: Hoffmann Eitle

(57) **Abstract**

Provided is an apparatus, including a first filtration unit that filters a fluid sample by a first filter; a culture unit for immersing the first filter after filtration by the first filtration unit in a liquid medium and performs culture; a second filtration unit that filters the liquid medium after culture by a partial region of the first filter; an extraction unit that extracts nucleic acids of organisms contained in the liquid medium from a residue after filtration by the second filtration unit; and a production unit that produces a second filter by cutting out the partial region of the first filter immersed in the liquid medium, wherein the second filtration unit filters the liquid medium after culture by the second filter.

## Description

### BACKGROUND

### 1. TECHNICAL FIELD

The present invention relates to an apparatus and a method.

### 2. RELATED ART

In Patent Document 1 to 5, it is described that "microorganisms are collected on a membrane filter with an outer frame, and the microorganisms are cultured, immobilized, hybridized with a fluorescent probe, washed, and observed with a fluorescence microscope while the microorganisms are maintained on the membrane filter with the outer frame".

### Prior Art Documents

### Patent Document

Patent Document 1: Japanese Patent Application Publication No. 2006-296285
Patent Document 2: Japanese Patent Application Publication No. 2007-202553
Patent Document 3: Japanese Patent Application Publication No. 2009-034093
Patent Document 4: Japanese Patent Application Publication No. 2010-213724
Patent Document 5: Japanese Patent Application Publication No. 2009-082153
Patent Document 6: International Publication No. 2019/43779
Patent Document 7: Japanese Patent No. 5624487
Patent Document 8: Japanese Patent No. 4857373

### SUMMARY

A first aspect of the present invention provides an apparatus including: a first filtration unit that filters a fluid sample by a first filter; a culture unit that performs culture by immersing the first filter after filtration by the first filtration unit in a liquid medium; a second filtration unit that filters the liquid medium after culture by a partial region of the first filter; and an extraction unit that extracts nucleic acids of organisms contained in the liquid medium from a residue after filtration by the second filtration unit.

The above-described apparatus may further include a production unit that produces a second filter by cutting out the partial region of the first filter immersed in the liquid medium. The second filtration unit may filter the liquid medium after culture by the second filter. The first filter immersed in the liquid medium may be picked up from the liquid medium after completion of culture, or may be picked up from the liquid medium during a period from the start to the completion of the culture. The production unit may produce a second filter by cutting the first filter in a wet state due to the liquid medium, or may produce a second filter by drying and cutting the first filter.

In any of the above-described apparatus, a cross-sectional shape of a flow channel in the second filtration unit may be smaller than a cross-sectional shape of a flow channel in the first filtration unit.

In any of the above-described apparatus, the extraction unit may contain and heat the second filter that has filtered the liquid medium after culture to extract nucleic acids of organisms in a sealed container.

Any of the above-described apparatus may further include a third filtration unit that filters a fluid sample by a pre-filter with meshes larger than those of the first filter before filtration by the first filtration unit.

A second aspect in the present invention provides a method including first filtering for filtering a fluid sample by a first filter; culturing for immersing the first filter after filtration at the first filtering in a liquid medium and performing culture; second filtering for filtering the liquid medium after culture by a partial region of the first filter; and extracting nucleic acids of organisms contained in the liquid medium from a residue after filtration at the second filtering.

The summary of the above-described invention does not necessarily describe all necessary features of the embodiments of the present invention. The present invention may also be a sub-combination of the feature groups described above.

### BRIEF DESCRIPTION OF THE DRAWINGS

Fig. 1 illustrates a microorganism detection apparatus 1 according to an embodiment.
Fig. 2 illustrates states of a first filter 101 and a second filter 102 in a first filtration unit 12, a culture unit 13, a production unit 14, a second filtration unit 15 and a nucleic acid extraction unit 16.
Fig. 3 illustrates operations of the microorganism detection apparatus 1.

### DESCRIPTION OF EXEMPLARY EMBODIMENTS

Hereinafter, the invention will be described through embodiments of the invention, but the following embodiments do not limit the invention according to claims. In addition, not all of the combinations of features described in the embodiments are essential to the solving means of the invention.

### < 1. Microorganism Detection Apparatus>

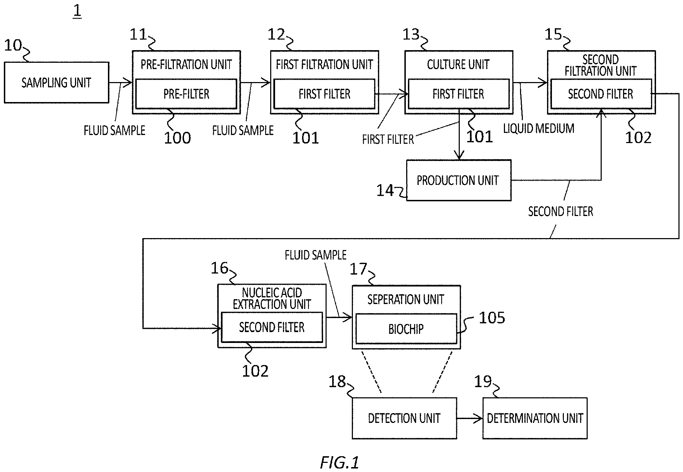

Fig. 1 illustrates a microorganism detection apparatus 1 according to the present embodiment. The microorganism detection apparatus 1 may be an example of an apparatus and detects microorganisms mixed in food and beverages. The microorganism detection apparatus 1 includes a sampling unit 10, a pre-filtration unit 11, a first filtration unit 12, a culture unit 13, a production unit 14, a second filtration unit 15, a nucleic acid extraction unit 16, a separation unit 17, a detection unit 18 and a determination unit 19.

The microorganisms to be detected can be selected from a group consisting of, as an example, Acintobacter species, Actinomyces species, Aerococcus species, Aeromonas species, Alclaigenes species, Bacillus species, Bacteriodes species, Bordetella species, Branhamella species, Brevibacterium species, Campylobacter species, Candida species, Capnocytophaga species, Chromobacterium species, Clostridium species, Corynebacterium species, Cryptococcus species, Deinococcus species, Enterococcus species, Erysipelothrix species, Escherichia species, Flavobacterium species, Gemella species, Haemophilus species, Klebsiella species, Lactobacillus species, Lactococcus species, Legionella species, Leuconostoc species, Listeria species, Micrococcus species, Mycobacterium species, Neisseria species, Cryptosporidium species, Nocardia species, Oerskovia species, Paracoccus species, Pediococcus species, Peptostreptococcus species, Propionibacterium species, Proteus species, Pseudomonas species, Rahnella species, Rhodococcus species, Rhodospirillium species, Staphlococcus species, Streptomyces species, Streptococcus species, Vibrio species and Yersinia species. There are microorganisms that take the form of blasts and spores in oligotrophic conditions, regardless of the cellular status due to such growth conditions.

### <1.1. Sampling Unit 10>

The sampling unit 10 extracts a sample from food or a beverage. The sampling unit 10 may extract a sample by suctioning the beverage or may extract a sample by suctioning the beverage and filtering the beverage through a filter (also referred to as a membrane filter). The sampling unit 10 may crush the food to extract a sample. The sampling unit 10 may supply the sample as a fluid sample to the pre-filtration unit 11. When extracting the sample obtained by crushing the food, the sampling unit 10 may supply the sample as a fluid sample in saline or liquid medium to the pre-filtration unit 11.

### <1.2. Pre-filtrationUnit 11>

The pre-filtration unit 11 is an example of a third filtration unit, and filters the fluid sample with a pre-filter 100 before filtration by the first filtration unit 12. The pre-filter 100 may have larger meshes (also referred to as filter pore size) than the first filter 101 described below, which is used in the first filtration unit 12. For example, the pre-filtration unit 11 may coarsely filter the fluid sample, and the pre-filter 100 may have larger meshes than 10 µm. The pre-filter 100 may remove foreign substances other than the microorganisms from the fluid sample. The material of the pre-filter 100 is not limited particularly as long as the material does not interfere with filtration in the first filtration unit 12 or culture in the culture unit 13. The material of the pre-filter 100 may use any of the materials described as the material of the first filter 101 described below. The pre-filtration unit 11 may supply the fluid sample after filtration to the first filtration unit 12.

### <1.3. First Filtration Unit 12>

The first filtration unit 12 filters the fluid sample by the first filter 101.

The first filter 101 used for filtration preferably has a pore size, by which microorganisms to be detected are to be captured. For example, the first filtration unit 12 may microfilter the fluid sample, and the first filter 101 preferably have meshes of 0.45 µm or less. The first filter 101 may be a screen filter or a depth filter, but in the present embodiment it is a screen filter as an example. The planar shape of the first filter 101 may be the same as the cross-sectional shape of the flow channel in the first filtration unit 12, as long as the first filter 101 is not flushed by the fluid sample in the first filtration unit 12, and may be circular as an example.

The material of the first filter 101 is not particularly limited as long as the material does not interfere with the extraction of the nucleic acids in the nucleic acid extraction unit 16 described below and the extracted nucleic acids are not easily adsorbed. For example, the material of the first filter 101 may be polytetrafluoroethylene (PTFE), polyvinylidene fluoride (PVDF), polyethersulfone (PES), cellulose mixed ester, polycarbonate (PC), nylon, polyvinyl chloride (PVC), pure silver, etc. The term "cellulose mixed ester" is a biologically inert material consisting of a mixture of cellulose acetate and cellulose nitrate. The material of the first filter 101 is preferably hydrophilic, for example, hydrophilic PTFE, hydrophilic PVDF, hydrophilic PES or hydrophilic PC preferably. When the first filter 101 is hydrophilic, nucleic acids extracted in the nucleic acid extraction unit 16 described below are not easily adsorbed on the first filter 101. Among the materials, hydrophilic PC is more preferred as the material of the first filter 101. When the material of the first filter 101 is hydrophilic PC, the pore size or the pore size distribution tends to be constant. By using the first filter 101 with constant pore size or pore size distribution, microorganisms can be collected stably. One indicator indicative of the hydrophilic nature of the material of the first filter 101 can be the contact angle as an example. For example, a filter made by Merck Corporation (Millipore Corporation) or a filter made by Advantech Toyo Corporation can be used as the above filter. The first filtration unit 12 may supply the first filter 101 with microorganisms captured as a residue to the culture unit 13.

### <1.4. Culture Unit 13>

The culture unit 13 immerses the first filter 101 after filtration by the first filtration unit 12 in the liquid medium and performs culture. The culture unit 13 may culture the microorganisms captured by the first filter 101. The culture unit 13 may perform culture under any conventionally well known method or conditions according to the microorganisms to be detected. The liquid medium may be a solution of dissolved solid medium.

The culture unit 13 may supply the liquid medium after culture to the second filtration unit 15. The culture unit 13 may supply the first filter 101 that has been immersed in the liquid medium to the production unit 14. As an example, the culture unit 13 may pick up the first filter 101 from the liquid medium after completion of culture to supply it to the production unit 14, or may pick up the first filter 101 from the liquid medium during a period from the start to the completion of culture to supply it to the production unit 14.

### <1.5. Production Unit 14>

The production unit 14 cuts out a partial region of the first filter 101 immersed in the liquid medium to produce a second filter 102. The production unit 14 may produce the second filter 102 from the first filter 101 supplied from the culture unit 13. The production unit 14 may cut the first filter 101 in a wet state due to the liquid medium to produce the second filter 102, or may dry then cut the first filter 101 to produce the second filter 102. The production unit 14 may discard the remaining portion of the first filter 101 that was not used as the second filter 102. When the first filter 101 is picked up from the liquid medium during a period from the start to the completion of the culture by the culture unit 13 and supplied to the production unit 14, the production unit 14 may return the remaining portion of the supplied first filter 101 that was not used as the second filter 102 to the culture unit 13 for re-immersion in the liquid medium.

The production unit 14 may produce the second filter 102 according to the cross-sectional shape of the flow channel of the second filtration unit 15. As an example, the production unit 14 may produce the second filter 102 by cutting the first filter 101 by using a mold preformed to match the cross-sectional shape of the flow channel of the second filtration unit 15. The cross-sectional shape of the flow channel in the second filtration unit 15 may be smaller than the cross-sectional shape of the flow channel in the first filtration unit 12, and may be smaller than the planar shape of the first filter 101. The planar shape of the second filter 102 may be the same as the cross-sectional shape of the flow channel of the second filtration unit 15, as long as the second filter 102 is not flushed by the fluid sample in the second filtration unit 15, and may be circular as an example. The production unit 14 may supply the produced second filter 102 to the second filtration unit 15.

### <1.6. Second Filtration Unit 15>

The second filtration unit 15 filters the liquid medium after culture by a partial region of the first filter 101. In this manner, the cultured microorganisms are collected as a residue. The second filtration unit 15 may filter the liquid medium after culture by the second filter 102 produced by the production unit 14.

The second filtration unit 15 may supply the collected microorganisms to the nucleic acid extraction unit 16. In the present embodiment, the second filtration unit 15 may supply the microorganisms collected as a residue on the second filter 102 to the nucleic acid extraction unit 16 with the entire second filter 102 as an example.

### <1.7. Nucleic Acid Extraction Unit 16>

The nucleic acid extraction unit 16 extracts nucleic acids of the microorganisms contained in the liquid medium (as an example, genomic DNA, ribosomal RNA, plasmid DNA, etc.) from the residue after filtration by the second filtration unit 15. The nucleic acid extraction unit 16 may contain and heat the second filter 102 that has filtered the liquid medium after culture in a container that is sealed (also referred to as a sealed container) and extract nucleic acids of the microorganisms, and may extract nucleic acids by using a so-called dHTP method, which is a method according to, for example, International Publication No. 2019/43779 or Japanese Patent No. 5624487.

The nucleic acid extraction unit 16 may expose microorganisms as a residue to high temperature conditions with the entire second filter 102 in the sealed container to destroy the membrane structures of microorganisms and make the nucleic acids in an extractable state. The container may be, for example, a thermally fusible bag or a boil lock tube. The container may be, for example, a plastic tube, a glass test tube, or a microfluidic chip.

The container may be heated by heating means not shown. The heating means may be an oil bath or a heat block, which may heat the inside of the container to a temperature of up to 200°C. The pressure in the container may become atmospheric pressure or higher due to heating.

At least one cell dissolution promoter selected from the group consisting of an alkali, acid, enzyme, surfactant, redox agent or protein denaturing agent having the ability to dissolve membrane structures may be added into the container.

For example, sodium hydroxide (NaOH), potassium hydroxide (KOH) or the like can be used as the above-described alkali. For example, hydrochloric acid (HCl), sulfuric acid (H₂SO₄) or the like can be used as the above-described acid. For example, a proteinase enzyme such as Proteinase K, or a polysaccharide-degrading enzyme such as chitinase, lysozyme, zymolyase, etc. can be used as the above-described enzyme. The above-described surfactant may be ionic or nonionic, for example. For example, octylphenol ethoxylate (C₁₄H₂₂O(C₂H₄O)ₙ) or the like can be used as the nonionic surfactant. For example, commercial products such as TritonX-100 (C₁₄H₂₂O(C₂H₄O)ₙ, n=100) made by SIGMA Corporation can be used as the octylphenol ethoxylate.

The ionic surfactant may be anionic, may be cationic, or may be amphionic. For example, sodium dodecyl sulfate (SDS) or the like can be used as an anionic surfactant. For example, cetyltrimethylammonium bromide (CATB) or the like can be used as a cationic surfactant. For example, betaine or the like can be used as an amphionic surfactant. Herein, "betaine" is a generic term for compounds that have a positive charge and a negative charge at non-adjacent positions in the same molecule, have no dissociable hydrogen atom bonded to the atom with a positive charge, and have no charge in the molecule as a whole. Trimethylglycine can be used as a typical example of betaine. For example, hydrogen peroxide, β-mercaptoethanol, dithiothreitol or the like can be used as the above-described redox agent.

For example, guanidine hydrochloride, urea or the like can be used as the above-described protein denaturing agent. For example, ethylenediaminetetraacetic acid (EDTA) or the like can be used as the above-described chelating agent. Among the above-mentioned cell dissolution promoters, it is preferred that the above-described surfactant is added, and it is more preferred that either one or both of SDS and octylphenol ethoxylate are added. For example, when nucleic acids to be extracted is to be detected with high sensitivity, SDS may be used. In contrast, when the extracted nucleic acids are to be used for enzymatic reactions that are inhibited by SDS, octylphenol ethoxylate, which acts more mildly than SDS on membrane structures of microorganisms, may be used.

The container may further contain a buffer solution inside. For example, tris (hydroxymethyl) aminomethane hydrochloride (Tris-HCl) or the like can be used as the buffer solution.

The nucleic acid extraction unit 16 may extract nucleic acids from the liquid inside the container after the membrane structures of microorganisms have been destroyed. The nucleic acid extraction unit 16 may bond nucleic acids with magnetic microparticles to extract the nucleic acids by magnetism by, for example, the method according to Japanese Patent No. 4857373. Alternatively, the nucleic acid extraction unit 16 may extract nucleic acids by at least one of filtration, centrifugation or electrophoresis. When nucleic acids are extracted by electrophoresis, the nucleic acids may be charged beforehand by well known methods.

The nucleic acid extraction unit 16 may supply the fluid sample containing the extracted nucleic acids to the separation unit 17. The nucleic acid extraction unit 16 may supply the fluid sample with amplified nucleic acids to the separation unit 17. The nucleic acid extraction unit 16 may amplify nucleic acids in the liquid in a state where the membrane structures of microorganisms has been destroyed, or may extract and then amplify the nucleic acids after extracting the nucleic acids. The nucleic acid extraction unit 16 may amplify nucleic acids by the method of polymerase chain reaction (PCR).

### <1.8. Separation Unit 17>

The separation unit 17 separates nucleic acids of the microorganisms to be detected from the nucleic acids in the fluid sample. The separation unit 17 may separate nucleic acids that are unique to the microorganisms to be detected (also referred to as target nucleic acids) from the nucleic acids of the microorganisms to be detected. The separation unit 17 may separate the target nucleic acids using the biochip 105.

The biochip 105 may have a plurality of probes that each hybridizes with a corresponding base sequence. At least some of the plurality of probes may have a base sequence complementary to at least a portion of the base sequence of the target nucleic acid and may specifically hybridize with the nucleic acid of the corresponding base sequence. At least some of the plurality of probes may have the same base sequence.

Each probe may be pre-fixed at a unique position within the biochip 105. As an example in the present embodiment, the biochip 105 may have a plurality of probes on the inner surfaces of two transparent substrates arranged opposite each other. The biochip 105 may have an inlet for implanting a fluid sample and an outlet for draining the fluid sample inside.

At least one of the probes of the biochip 105 having a complementary base sequence or the nucleic acids in the fluid sample may be pre-attached with a label that emits a signal detectable by the detection unit 18 described below in a hybridized state. For example, the label may be attached to the probe and may emit a signal according to the hybridization between the probe and the nucleic acids. Alternatively, the label may be attached to nucleic acids in the fluid sample and may emit the signal regardless of whether the nucleic acids hybridize with the probe. In this case, after the implantation of the fluid sample into the biochip 105, nucleic acids that have not hybridized with the probe may be removed from the biochip 105, and then the signal may be detected. A fluorescent dye, a radioisotope or a paramagnetic isotope, an enzyme or the like can be used as the label. As an example in the present embodiment, the label may be a fluorescent dye, which may be attached to the probe.

### <1.9. Detection Unit 18>

The detection unit 18 detects the label inside the biochip 105. The detection unit 18 may be disposed opposite the biochip 105 and may detect the label inside the biochip 105 via the transparent substrate of the biochip 105. The detection unit 18 may detect a signal emitted from a label attached to either one of the hybridized probes or the nucleic acid. The detection unit 18 may supply information indicating the position of the detected label, or the intensity of the signal emitted from the label (fluorescence intensity as an example in the present embodiment) to the determination unit 19.

### <1.10. Determination Unit 19>

The determination unit 19 determines whether the fluid sample implanted into the biochip 105 contained more nucleic acids of microorganisms to be detected than the reference value, or in other words, whether the fluid sample was any of positive or negative. The determination unit 19 may perform determination based on the information supplied from the detection unit 18. For example, the determination unit 19 may perform determination according to a detected position of the label by the detection unit 18. The determination unit 19 may output the determination result indicating whether the fluid sample was any of positive or negative to the outside.

The reference value may indicate the number of nucleic acids, and may be a number of labels as target nucleic acids detected by the detection unit 18. As an example in the present embodiment, the reference value may be 0. However, the reference value may indicate a proportion of nucleic acids, or may be a proportion of the labels detected by the detection unit 18 as the target nucleic acids among the number of labels detected by the detection unit 18.

According to the microorganism detection apparatus 1 described above, organisms can be extracted from the liquid medium after culture using a partial region of the first filter 101 for filtering organisms contained in the fluid sample, thus being capable of reducing the usage amount of filters compared to the case of using separate filters before and after culture.

Because the second filter 102 of the second filtration unit 15, which filters the liquid medium, is produced by cutting out a partial region of the first filter 101, a second filter 102 compatible with the second filtration unit 15 can be produced from the first filter 101 used in the first filtration unit 12. Therefore, filtration can be reliably performed in the second filtration unit 15.

The cross-sectional shape of the flow channel in the second filtration unit 15 is smaller than the cross-sectional shape of the flow channel in the first filtration unit 12, so a partial region of the first filter 101 can be used to reliably perform filtration in the second filtration unit 15.

The nucleic acids of microorganisms are extracted by containing and heating the second filter 102 that has filtered the liquid medium after culture in a sealed container, thus capable of eliminating the need to separate the microorganisms from the second filter 102 and making it easier to obtain the nucleic acids of the microorganisms in the liquid sample.

Because the fluid sample is filtered by the pre-filter 100, which has meshes larger than those of the first filter 101 before filtration by the first filtration unit 12, foreign substances in the fluid sample can be removed for performing culture.

### <2. First Filter 101 and Second Filter 102>

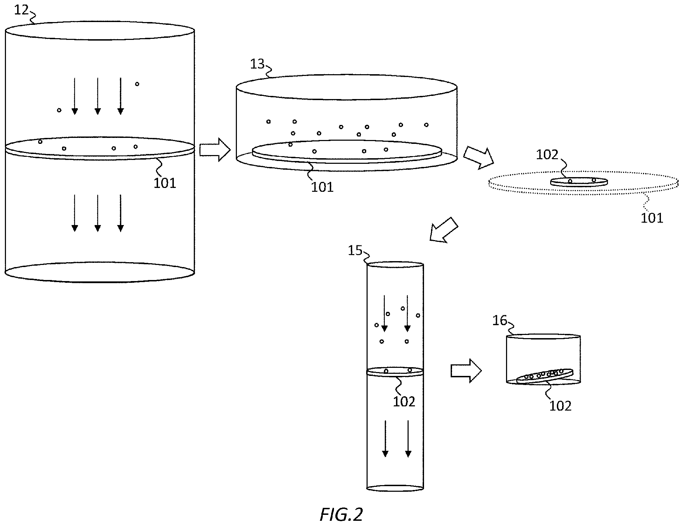

Fig. 2 illustrates the state of the first filter 101 and the second filter 102 in the first filtration unit 12, the culture unit 13, the production unit 14, the second filtration unit 15 and the nucleic acid extraction unit 16. The white circle symbols in the figure indicate microorganisms to be detected. The white arrow symbols in the figure indicate the process flow in the microorganism detection apparatus 1.

The first filter 101 is used to filter the fluid sample in the first filtration unit 12 to capture microorganisms (see upper left in the figure), and then immersed in the liquid medium in the culture unit 13 (see upper center in the figure). Then, the first filter 101 is processed by the production unit 14 to become the second filter 102 (see upper right in the figure), which is then used to filter the liquid medium in the second filtration unit 15 to capture microorganisms (see lower center in the figure). In the second filter 102 is contained in a sealed container in the nucleic acid extraction unit 16 and heated (see lower right in the figure), thereby extracting the nucleic acids of the microorganisms contained in the sample.

### <3. Operations of Microorganism Detection Apparatus 1>

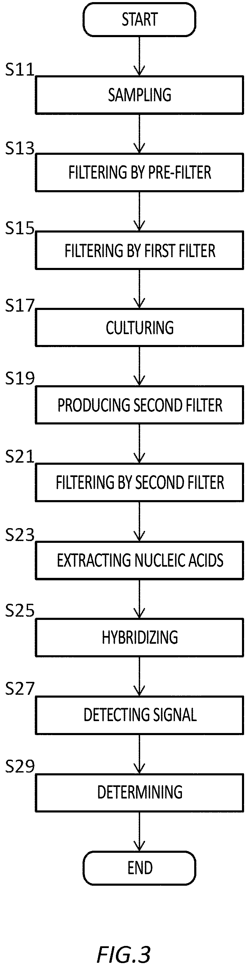

Fig. 3 illustrates operations of the microorganism detection apparatus 1. By performing the process of steps S11 to S29, the microorganism detection apparatus 1 determines whether each sample contains more microorganisms to be detected than the reference value.

In step S11, the sampling unit 10 extracts samples from food and beverages. The sampling unit 10 may produce a fluid sample from the sample.

In step S13, the pre-filtration unit 11 filters the fluid sample by the pre-filter 100. This may remove foreign substances from the fluid sample.

In step S15, the first filtration unit 12 filters the fluid sample by the first filter 101. This may capture the microorganisms to be detected as a residue.

In step S17, the culture unit 13 immerses the first filter 101 after filtration in the liquid medium to perform culture. Microorganisms may be captured in the first filter 101. Thereby the microorganisms captured in the first filter 101 are cultured.

In step S19, the production unit 14 cuts out a partial region of the first filter 101 immersed in the liquid medium to produce the second filter 102. The second filter 102 may have microorganisms captured in the first filter 101 in step S15 and microorganisms grown in step S17 attached thereto. The production unit 14 may be washed after the production of the second filter 102. This prevents the microorganisms attached to the first filter 101 from remaining in the production unit 14.

In step S21, the second filtration unit 15 filters the liquid medium after culture by a partial region of the first filter 101 (in the present embodiment, the second filter 102 as an example). This may capture the microorganisms cultured in step S17. The liquid medium filtered by the second filter 102 in step S21 may be the liquid medium in which the first filter 101, from which the second filter 102 is derived, was immersed in step S17.

In step S23, the nucleic acid extraction unit 16 extracts the nucleic acids of the microorganisms contained in the liquid medium from the residue after filtration by the second filtration unit 15. As an example, the nucleic acid extraction unit 16 may contain the microorganisms as a residue in a container with the entire second filter 102, destroy the membrane structures of microorganisms using the so-called dHTP method, and then extract the nucleic acids by filtration or centrifugation or the like.

In step S25, the separation unit 17 separates nucleic acids of the microorganisms to be detected from the nucleic acids in the fluid sample. As an example, the separation unit 17 may separate the nucleic acids of the microorganisms to be detected contained in the fluid sample by hybridizing them with the probe of the biochip 105.

In step S27, the detection unit 18 detects the label inside the biochip 105. As an example, the detection unit 18 may detect a signal emitted from the label attached to at least one of the probes of the biochip 105 or the nucleic acids in the fluid sample.

In step S29, the determination unit 19 determines whether the fluid sample implanted into the biochip 105 contained more nucleic acids of the microorganisms to be detected than the reference value, or in other words, whether the fluid sample was any of positive or negative, based on information supplied from the detection unit 18.

### <4. Modification Example>

In the above-described embodiment, the microorganism detection apparatus 1 is described as including a sampling unit 10, a pre-filtration unit 11, a production unit 14, a separation unit 17, a detection unit 18, a determination unit 19, but any of these may not be included. For example, when the microorganism detection apparatus 1 does not include a production unit 14, the second filtration unit 15 may cover the entire cross-sectional area of the flow channel of the second filtration unit 15 with a partial region of the first filter 101, thereby filtering the liquid medium in the partial region. As an example, the second filtration unit 15 may be provided with tubular tangential portions that contact each other along the flow direction of the liquid medium, and by sandwiching the first filter 101 between these tangential portions, the entire cross-sectional area of the flow channel of the second filtration unit 15 may be covered with a partial region of the first filter 101.

Although the production unit 14 is described as producing the second filter 102 from the first filter 101 that has been used to filter the liquid sample and immersed in the liquid medium, the second filter 102 may be produced from the first filter 101 that has not been immersed in the liquid medium after being used to filter the liquid sample. In this case, the culture unit 13 may perform the culture by immersing the remaining portion of the first filter 101, of the first filter 101, which was not used as the second filter 102 in the liquid medium.

The determination unit 19 is described as determining whether the sample contains nucleic acids of the microorganisms to be detected, but it may also determine whether the sample contains nucleic acids of organisms other than the microorganisms. In this case, the culture unit 13 may culture cells of the organisms to be detected, and the second filtration unit 15 may collect the cultured cells. The organisms to be detected may be animals, insects, plants, mycoplasmas, viruses or the like. The organisms to be detected may be of one species, or may be of a plurality of species.

While the embodiments of the present invention have been described, the technical scope of the invention is not limited to the above described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by an apparatus, system, program, and method shown in the claims, embodiments, or diagrams can be performed in any order as long as the order is not indicated by "prior to," "before," or the like and as long as the output from a previous process is not used in a later process. Even if the process flow is described using phrases such as "first" or "next" in the claims, embodiments, or diagrams, it does not necessarily mean that the process must be performed in this order.

### EXPLANATION OF REFERENCES

1: microorganism detection apparatus; 10: sampling unit; 11: pre-filtration unit; 12: first filtration unit; 13: culture unit; 14: production unit; 15: second filtration unit; 16: nucleic acid extraction unit; 17: separation unit; 18: detection unit; 19: determination unit; 100: pre-filter; 101: first filter; 102: second filter; 105: biochip.

## Claims

1. An apparatus comprising:
a first filtration unit that filters a fluid sample by a first filter;
a culture unit that performs culture by immersing the first filter after filtration by the first filtration unit in a liquid medium;
a second filtration unit that filters the liquid medium after culture by a partial region of the first filter; and
an extraction unit that extracts nucleic acids of organisms contained in the liquid medium from a residue after filtration by the second filtration unit.

2. The apparatus according to claim 1, further comprising
a production unit that produces a second filter by cutting out the partial region of the first filter immersed in the liquid medium,
wherein the second filtration unit filters the liquid medium after culture by the second filter.

3. The apparatus according to claim 1 or 2, wherein a cross-sectional shape of a flow channel in the second filtration unit is smaller than a cross-sectional shape of a flow channel in the first filtration unit.

4. The apparatus according to claim 2, wherein the extraction unit contains and heats the second filter that has filtered the liquid medium after culture to extract nucleic acids of organisms in a sealed container.

5. The apparatus according to any one of claims 1 to 4, further comprising a third filtration unit that filters a fluid sample by a pre-filter with meshes larger than those of the first filter before filtration by the first filtration unit.

6. A method comprising:
first filtering for filtering a fluid sample by a first filter;
culturing for immersing the first filter after filtration at the first filtering in a liquid medium and performing culture;
second filtering for filtering the liquid medium after culture by a partial region of the first filter; and
extracting nucleic acids of organisms contained in the liquid medium from a residue after filtration at the second filtering.
